# EUROPEAN PATENT APPLICATION

(11) **EP 3 895 681 A1**
(43) Date of publication of application: **20.10.2021**
(21) Application number: 20745893.6
(22) Date of filing: 17.01.2020
(51) Int. Cl.: A61K 6/30, A61K 6/887, C08F 4/40, C09K 3/00, C08F 2/50

(54) **PHOTOPOLYMERIZATION INITIATOR, PHOTOCURABLE COMPOSITION, CURED PRODUCT, AND DENTAL MATERIAL**

(30) Priority: 21.01.2019 JP 2019007972; 28.03.2019 JP 2019064404
(71) Applicant: Mitsui Chemicals, Inc., Minato-ku Tokyo 105-7122 (JP)
(72) Inventor: TAKAHASHI, Issei, Sodegaura-shi, Chiba 299-0265 (JP)
(74) Representative: Hoffmann Eitle
(86) International application number: PCT/JP2020/001570
(87) International publication number: WO 2020/153269

(57) **Abstract**

A photopolymerization initiator including a peroxide, a photobase generator, and a photoradical generator; a photocurable composition; a cured product; and a dental material.

## Description

### TECHNICAL FIELD

The present disclosure relates to a photopolymerization initiator, a photocurable composition, a cured product, and a dental material.

### BACKGROUND ART

In the dental field, synthetic resin molded products have been used to repair defectives of teeth. For example, for repairing large defectives of teeth, curable compositions so-called composite resins have been widely used as teeth alternatives for a long time. In recent years, the application range of composite resins has been expanding.

Photopolymerization initiators are often used in polymerization of dental curable compositions such as composite resins. Photoradical generators such as camphorquinone (hereinafter, also referred to as "CQ"), photobase generators, and the like can often be included in the photopolymerization initiators. Among them, a combination of CQ and tertiary amine is one of the embodiments that are widely used in dental photopolymerization initiators.

For example, Japanese Patent Application Laid-Open (JP-A) No. 2016-8211 discloses a combination of α/diketone such as camphorquinone (CQ) and tertiary amine such as ethyl N, N-dimethylaminobenzoate, as an example of photopolymerization initiator that is used in a dental restorative material composition such as the above dental composite resin (curable composition).

### SUMMARY OF INVENTION

### Technical Problem

When the dental curable composition is used in the composite resin, depending on where it is to be applied, the composite resin is filled into a deep position of a tooth because of a deep damage of the tooth in some cases. In such a case, it is considered that favorably polymerizing the composite resin at the deep position of the tooth (that is, a high depth of polymerization) is important.

However, it is considered that the photopolymerization initiator material in which camphorquinone (CQ) and tertiary amine are used in combination as described in Japanese Patent Application Laid-Open (JP-A) No. 2016-8211 is insufficient in terms of a depth of polymerization.

An object of the present disclosure is to provide a photopolymerization initiator that is capable of improving a depth of polymerization of a photocurable composition, a photocurable composition including the photopolymerization initiator, a cured product, and a dental material.

### Solution to Problem

Solutions for the above object include the following embodiments.
<1> A photopolymerization initiator, comprising:
   a peroxide;
   a photobase generator; and
   a photoradical generator.
<2> The photopolymerization initiator according to <1>, wherein the photoradical generator is excited by light having a wavelength of from 420 nm to 525 nm.
<3> The photopolymerization initiator according to <1> or <2>, wherein the photoradical generator is a Norrish type II initiator, or an aniline compound and an iodonium salt compound.
<4> The photopolymerization initiator according to any one of <1> to <3>, wherein the peroxide comprises at least one selected from the group consisting of diacyl peroxide compounds, peroxyester compounds, hydroperoxide compounds, inorganic peroxides, and alkyl peroxides.
<5> The photopolymerization initiator according to any one of <1> to <4>, wherein the photobase generator comprises at least one selected from the group consisting of oxime ester compounds, acyl compounds, carbamate compounds, aminoacetophenone compounds, tertiary amine compounds, quaternary ammonium salt compounds, and amide compounds.
<6> The photopolymerization initiator according to any one of <1> to <5>, further comprising a benzodioxole compound.
<7> The photopolymerization initiator according to any one of <1> to <6>, which is for a dental material.
<8> A photocurable composition, comprising:
   the photopolymerization initiator according to any one of <1> to <7>; and
   a polymerizable monomer.
<9> The photocurable composition according to <8>, wherein a mass content ratio of the photoradical generator to a total amount of the polymerizable monomer is from 0.0001 to 1.0.
<10> The photocurable composition according to <8> or <9>, wherein a mass content ratio of the photobase generator to a total amount of the polymerizable monomer is from 0.0001 to 5.0.
<11> The photocurable composition according to any one of <8> to <10>, wherein a mass content ratio of the peroxide to a total amount of the polymerizable monomer is from 0.001 to 5.0.
<12> The photocurable composition according to any one of <8> to <11>, further comprising a filler.
<13> The photocurable composition according to <12>, wherein the filler comprises a filler having a relative permittivity of 8.0 or less at 25°C and 1 MHz.
<14> The photocurable composition according to <12> or <13>, wherein the filler comprises at least one filler selected from the group consisting of silica, barium borosilicate glass, and aluminosilicate glass.
<15> The photocurable composition according to any one of <8> to <14>, which is for a dental material.
<16> A cured product of the photocurable composition according to any one of <8> to <14>.
<17> A dental material, comprising the cured product according to <16>.

### Advantageous Effects of Invention

According to embodiments of the present disclosure, a photopolymerization initiator that is capable of improving a depth of polymerization of a photocurable composition, a photocurable composition including the photopolymerization initiator, a cured product, and a dental material can be provided.

### DESCRIPTION OF EMBODIMENTS

In the present disclosure, any numerical range expressed using "to" refers to a range that includes the numerical values indicated before and after "to" as the lower limit value and the upper limit value.

In the present disclosure, the amount of each component in a composition refers, in a case in which plural substances corresponding to each component are present in the composition, to the total amount of the plural substances present in the composition, unless otherwise specified.

In a numerical range described in stages in the present disclosure, the upper limit value or the lower limit value of one numerical range may be replaced with the upper limit value or the lower limit value of another numerical range described in stages. Further, in a numerical range described in the present disclosure, the upper limit value or the lower limit value of the numerical range may be replaced with a value shown in the Examples.

In the present disclosure, the term "(meth)acryloyl" refers to acryloyl or methacryloyl, and the term "(meth)acrylate" refers to acrylate or methacrylate.

In the present disclosure, each * in chemical formulae represents a binding position.

### «Photopolymerization Initiator»

The photopolymerization initiator of the present disclosure includes a peroxide, a photobase generator, and a photoradical generator.

For example, when filling and curing a composite resin in a deep position of a tooth, because light irradiation is performed from the surface side of the tooth, it is often difficult to polymerize and cure the composite resin in the deep position compared with a composite resin on the surface.

By including the photobase generator, the peroxide, and the photoradical generator, the photopolymerization initiator of the present disclosure is capable of generating a large number of radicals when the composite resin including the photopolymerization initiator is irradiated with light. Therefore, for example, polymerization of polymerizable monomers included in the composite resin can be favorably proceeded even in the deep position of a tooth.

Although the photopolymerization initiator of the present disclosure includes plural substances, it is referred to as a "photopolymerization initiator" or a "photopolymerization initiator composite material" for convenience.

### <Photoradical Generator>

The photopolymerization initiator of the present disclosure includes a photoradical generator.

The photopolymerization initiator of the present disclosure may be provided with only one kind of a photoradical generator, or may be provided with two or more kinds of photoradical generators.

The photoradical generator in the present disclosure is a photoradical generator other than the peroxides to be described later.

The photoradical generator in the photopolymerization initiator of the present disclosure is preferably a photoradical generator that is excited by light having a wavelength of from 420 nm to 525 nm.

This enables the photopolymerization initiator of the present disclosure to be more favorably used as a photopolymerization initiator included in a photocurable composition that is cured by light of visible region that is particularly less influential to bodies (for example, a region of wavelengths of from 420 nm to 525 nm). Specifically, the photopolymerization initiator of the present disclosure can be more favorably used as a medical photopolymerization initiator (particularly preferably as a photopolymerization initiator for a dental material).

Examples of the photoradical generator in the present disclosure include: acetophenones such as acetophenone, p-tert-butyltrichloroacetophenone, chloroacetophenone, 2,2-diethoxyacetophenone, hydroxyacetophenone, 2,2-dimethoxy-2'-phenylacetophenone, 2-aminoacetophenone, and 4'-methylaminoacetophenone; benzoins such as benzoin, benzoin methyl ether, benzoin ethyl ether, benzoin isopropyl ether, benzoin isobutyl ether, 1-hydroxycyclohexyl phenyl ketone, 2-hydroxy-2-methyl-1-phenyl-2-methylpropane-1-one, and 1-(4-isopropylphenyl)-2-hydroxy-2-methylpropane-1-one; benzophenones such as benzophenone, benzoylbenzoic acid, methyl benzoylbenzoate, methyl o-benzoylbenzoate, 4-phenylbenzophenone, hydroxybenzophenone, hydroxypropylbenzophenone, acrylbenzophenone, and 4,4'-bis(dimethylamino)benzophenone; thioxanthones such as thioxanthone, 2-chlorothioxanthone, 2-methylthioxanthone, diethylthioxanthone, and dimethylthioxanthone; α-acyloxime ester; benzyl-(o-ethoxycarbonyl)-α-monooxime; acylphosphine oxide; glyoxyester; 2-ethylanthraquinone; camphorquinone (CQ); camphorquinone; tetramethylthiuram sulfide; azobisisobutyronitrile; indane; and derivatives thereof; and an aniline compound and an iodonium salt compound to be described later.

Among them, because the photoradical generator is excited by light having a wavelength of from 420 nm to 525 nm, the photoradical generator of the present disclosure is preferably a Norrish type II photopolymerization initiator such as benzophenone, camphorquinone, indane, and derivatives thereof, or an aniline compound and an iodonium salt compound.

### (Norrish type II initiator)

When a Norrish type II photopolymerization initiator is used as the photoradical generator of the present disclosure, from a viewpoint of further improving a curing rate and a depth of polymerization of the photocurable composition, the Norrish type II photopolymerization initiator preferably includes at least one selected from the group consisting of benzophenone, camphorquinone, indane, and derivatives thereof, and more preferably includes camphorquinone.

### (Aniline Compound and Iodonium Salt Compound)

An aniline compound and an iodonium salt compound may be included as the photoradical generator in the present disclosure.

The photoradical generator in the present disclosure can be excited in a visible region by including an aniline compound and an iodonium salt compound. Furthermore, the color of a cured product of the photocurable composition can be suppressed.

### [Aniline Compound]

The photopolymerization initiator of the present disclosure may include a compound represented by the following Formula (1), as an aniline compound.

The photopolymerization initiator of the present disclosure may include only one kind of compound represented by the following Formula (1), or may include two or more kinds of compounds represented by the following Formula (1).

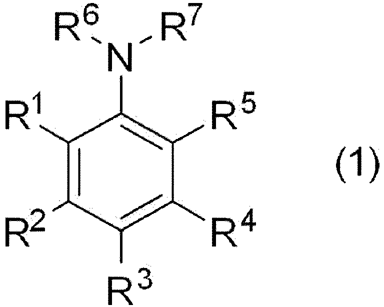

In Formula (1), R¹ to R⁷ each independently represent a hydrogen atom or a monovalent organic group having from 1 to 50 carbon atoms, wherein the organic group may include at least one substituent group selected from the group A consisting of an oxygen-containing group, a sulfur-containing group, a nitrogen-containing group, and a halogen atom. At least two of R¹ to R⁷ that are adjacent to one another may be bound to one another to form a ring.

In Formula (1), examples of the monovalent organic group having from 1 to 50 carbon atoms that is represented by R¹ to R⁷ include a monovalent hydrocarbon group, and a monovalent group including a hetero atom and a carbon atom.

Examples of the monovalent hydrocarbon group include an alkyl group, an alkenyl group, an alkynyl group, an aryl group, an alkylaryl group, an alkenylaryl group, an alkynylaryl group, an arylalkyl group, an arylalkenyl group, and an arylalkynyl group.

Examples of the hetero atom in the monovalent group including a hetero atom and a carbon atom include an oxygen atom, a sulfur atom, and a nitrogen atom, and at least one of an oxygen atom or a nitrogen atom is preferable.

In Formula (1), a carbon number in the monovalent organic group having from 1 to 50 carbon atoms that is represented by R¹ to R⁷ is preferably from 1 to 40, and more preferably from 1 to 30.

The monovalent organic group having from 1 to 50 carbon atoms that is represented by R¹ to R⁷ may include at least one substituent group selected from the group A.

The group A consists of an oxygen-containing group, a sulfur-containing group, a nitrogen-containing group, and a halogen atom.

In the group A, a fluorine atom, a chlorine atom, a bromine atom, or an iodine atom is preferable as the halogen atom.

In the group A, the oxygen-containing group is not particularly limited except that it is a group that contains an oxygen atom.

The oxygen-containing group may be a group consisting of an oxygen atom (that is, an oxo group (=O)).

A carbon number of the oxygen-containing group is preferably from 0 to 30, more preferably from 0 to 20, and more preferably from 0 to 10.

Examples of the oxygen-containing group include an oxo group (= O), a group represented by the following Formula (A), a group represented by the following Formula (B), a group represented by the following Formula (C), and a group represented by the following Formula (D).

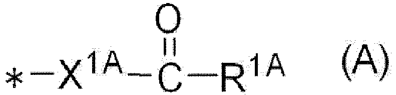

*-X^{1B}-OH (B)

*-X^{1C}-OH (C)

*-X^{1D}-O-R^{1D} (D)

In Formula (A), X^{1A} represents a single bond or a divalent hydrocarbon group having from 1 to 10 carbon atoms, R^{1A} represents a hydrogen atom, a monovalent hydrocarbon group having from 1 to 20 carbon atoms, or a monovalent organic group having from 3 to 30 carbon atoms, and * represents a binding position.

In Formula (B), X^{1B} represents a single bond or a divalent organic group having from 2 to 20 carbon atoms, and * represents a binding position.

In Formula (C), X^{1C} represents a single bond or a divalent organic group having from 1 to 20 carbon atoms, and * represents a binding position.

In Formula (D), X^{1D} represents a single bond or a monovalent organic group having from 1 to 20 carbon atoms, and * represents a binding position.

In the group A, the sulfur-containing group is not particularly limited except that it is a group that contains a sulfur atom. The sulfur-containing group may be a group consisting of a sulfur atom (that is, a thioxo group (=S)).

Examples of the sulfur-containing group include a thiol group (-SH) and a thioxo group (=S).

In the group A, the nitrogen-containing group is not particularly limited except that it is a group that contains a nitrogen atom. The nitrogen-containing group may be a group consisting of a nitrogen atom (that is, a nitrilo group (=N)).

Examples of the nitrogen-containing group include a nitrilo group (=N), an amino group (-NH2), a monoalkylamino group having from 1 to 10 carbon atoms, and a dialkylamino group having from 2 to 20 carbon atoms.

In Formula (1), R⁶ and R⁷ each independently preferably represent an alkyl group having from 1 to 4 carbon atoms, or an alkylene group having from 2 to 8 carbon atoms that is formed by binding R⁶ and R⁷ with each other (that is, a group for forming a nitrogen-containing heterocycle together with a nitrogen atom).

In Formula (1), when all of R¹ to R⁵ are hydrogen atoms, it is preferable that R⁶ and R⁷ each independently represent a methyl group or an ethyl group. In this case, it is more preferable that both R⁶ and R⁷ represent methyl groups or ethyl groups.

A molecular weight of the compound represented by Formula (1) is 600 or less, but it is not particularly limited.

Specific examples (compound (1-1) to compound (1-21)) of the compound represented by Formula (1) are shown below as particularly preferred examples; however, the compound represented by Formula (1) is not limited to the following specific examples.

The photopolymerization initiator of the present disclosure may include only one kind of these specific examples, or may include two or more kinds of these specific examples.

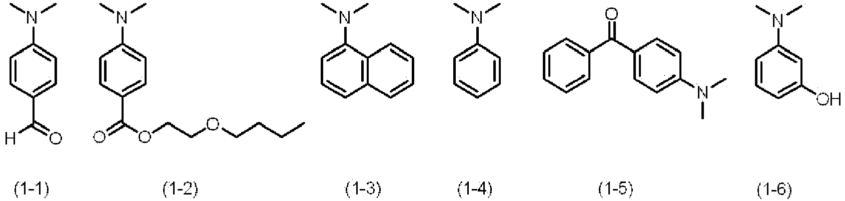

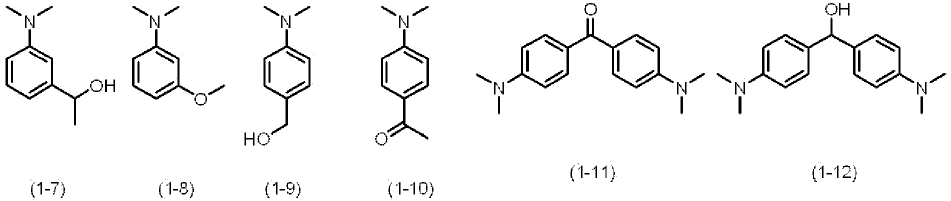

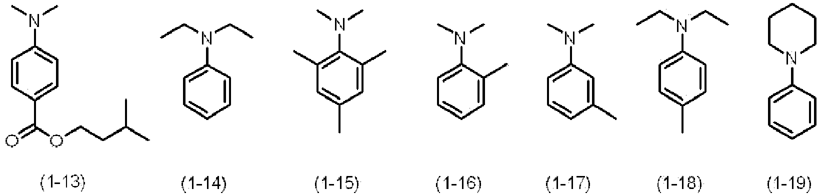

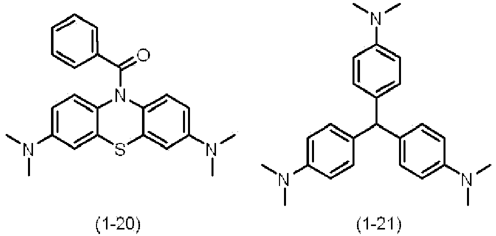

### [Iodonium Salt Compound]

The photopolymerization initiator of the present disclosure may include a compound represented by the following Formula (2), as an iodonium salt compound.

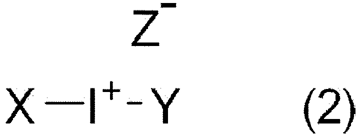

In Formula (2), X and Y each independently represent a monovalent hydrocarbon group having from 6 to 50 carbon atoms and including an aromatic ring, wherein the monovalent hydrocarbon group may include at least one substituent group selected from the group A consisting of an oxygen-containing group, a sulfur-containing group, a nitrogen-containing group, and a halogen atom. In Formula (2), I⁺ represents an iodine cation, and Z⁻ represents an anion.

The compound represented by Formula (2) is a salt that consists of a cation represented by X-I⁺-Y and an anion represented by Z⁻.

In Formula (2), at least one substituent group selected from the group A is the same as the at least one substituent group selected from the group A in Formula (1).

It is preferable that X and Y each independently represent an unsubstituted aryl group, an aryl group substituted with a halogen atom, or an aryl group substituted with an alkyl group.

The halogen atom is preferably a fluorine atom, a chlorine atom, a bromine atom, or an iodine atom.

The carbon numbers of X and Y are, each independently, preferably from 6 to 30, more preferably from 6 to 20, and still more preferably from 6 to 12.

In Formula (2), Z⁻ represents an anion.

The kind of the anion represented by Z⁻ is not particularly limited, and examples thereof include PF₆⁻, P(CF₃)₃F₃⁻, CF₃SO₃⁻, CI, CF₃C₃F₆SO₃, CH₃C₆H₄SO₃⁻, and NO₃⁻.

A molecular weight of the compound represented by Formula (2) is not particularly limited, for example, it may be 280 or more but 1000 or less.

Particularly preferred specific examples (compound (2-1) to compound (2-6)) of the compound represented by Formula (2) are shown below; however, the compound represented by Formula (2) is not limited to the following specific examples.

The photopolymerization initiator of the present disclosure may include only one kind of these specific examples, or may include two or more kinds of these specific examples.

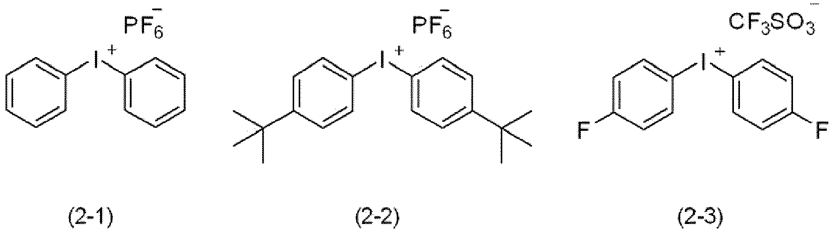

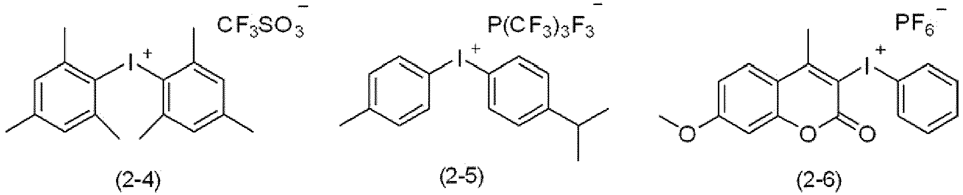

When the photopolymerization initiator of the present disclosure includes a compound represented by Formula (2) in addition to a compound represented by Formula (1), in the photopolymerization initiator of the present disclosure, a molar ratio of a compound represented by Formula (2) to a compound represented by Formula (1) (that is, a molar ratio [compound represented by Formula (2)/compound represented by Formula (1)]) is, for example, preferably from 0.3 to 2.

### <Complex>

In the photopolymerization initiator of the present disclosure, when an aniline compound and an iodonium salt compound are used as a photoradical generator, a complex of a compound represented by Formula (1) and a compound represented by Formula (2) is preferably formed.

The formation of a complex of a compound represented by Formula (1) and a compound represented by Formula (2) is confirmed as follows.

First, the following sample A, sample B, and sample C are prepared:
sample A consists of a compound represented by Formula (1), a compound represented by Formula (2), and triethyleneglycol dimethacrylate (TEGDMA);
sample B consists of a compound represented by Formula (1) and TEGDMA; and
sample C consists of a compound represented by Formula (2) and TEGDMA.

Here, volumes of sample A, sample B, and sample C are the same.

Further, a concentration of a compound represented by Formula (1) in sample A and a concentration of a compound represented by Formula (1) in sample B are adjusted so as to be the same value.

Furthermore, a concentration of a compound represented by Formula (2) in sample A and a concentration of a compound represented by Formula (2) in sample C are adjusted so as to be the same value.

Subsequently, ultraviolet-visible (UV-Vis) absorption spectrum (hereinafter, also simply referred to as a "spectrum") of each sample A, sample B, and sample C is measured.

When an area of a spectrum of sample A in a wavelength range of from 350 nm to 550 nm is larger than a total area of a spectrum of sample B in a wavelength range of from 350 nm to 550 nm and a spectrum of sample C in a wavelength range of from 350 nm to 550 nm, a complex of a compound represented by Formula (1) and a compound represented by Formula (2) is determined to have been formed in the photopolymerization initiator of the present disclosure.

A complex of a compound represented by Formula (1) and a compound represented by Formula (2) can be formed by mixing a compound represented by Formula (1) and a compound represented by Formula (2) (and, if necessary, other components). For example, the above complex as a photopolymerization initiator can be formed by mixing a compound represented by Formula (1), a compound represented by Formula (2), and a polymerizable monomer when preparing a photocurable composition to be described later.

A temperature of each component when performing the above mixing is, for example, from 20°C to 80°C.

A time of performing the above mixing is, for example, from 10 minutes to 5 hours.

Although a molar ratio of a compound represented by Formula (2) to a compound represented by Formula (1) (that is, a molar ratio [compound represented by Formula (2)/compound represented by Formula (1)]) when performing the above mixing is not particularly limited, it is preferably from 0.3 to 2.0, and more preferably from 0.5 to 1.5.

A content of a photoradical generator with respect to a total amount of the photopolymerization initiator of the present disclosure is preferably from 0.0001% by mass to 1.0% by mass, more preferably from 0.0001% by mass to 0.005% by mass, and still more preferably from 0.0002% by mass to 0.0008% by mass.

The above term "a total amount of the photopolymerization initiator" means a total mass of a photoradical generator, a peroxide, a photobase generator, and a reducing agent as an optional component. In this respect, the same applies hereinafter.

In the present disclosure, for example, a content of a photoradical generator can be selected as appropriate and used in consideration of the above content ratio [photoradical generator/polymerizable monomer], in a range of from 1.0% by mass to 99.0% by mass with respect to a total content of a peroxide, a photobase generator, and a photoradical generator.

### <Reducing Agent>

The photopolymerization initiator of the present disclosure may further include a reducing agent.

By the photopolymerization initiator of the present disclosure including a reducing agent, radicals can be generated more efficiently from a photoradical generator specifically when the photopolymerization initiator is irradiated with visible light.

The photopolymerization initiator of the present disclosure may be provided with only one kind of reducing agent, or may be provided with two or more kinds of reducing agents.

Examples of the reducing agent include amines and benzodioxole compounds.

Among them, from the viewpoint of stability of a photocurable composition including the photopolymerization initiator of the present disclosure, the photopolymerization initiator of the present disclosure preferably further includes a benzodioxole compound (specifically a benzodioxole compound that does not include a nitrogen atom) as a reducing agent.

The reason that stability of a photocurable composition including the photopolymerization initiator can be improved when a benzodioxole compound is used as a reducing agent is presumed as follows.

The photopolymerization initiator of the present disclosure includes a photoradical generator. When the photoradical generator and a reducing agent are used together, stability of a photocurable composition including the photopolymerization initiator of the present disclosure is impaired in some cases by a reaction of the reducing agent and a peroxide included in the photopolymerization initiator of the present disclosure.

However, when a benzodioxole compound is selected as a reducing agent that is to be used together with the photoradical generator, stability of the photocurable composition including the photopolymerization initiator of the present disclosure is well maintained because reactivity of the benzodioxole compound as a reducing agent with the peroxide is low.

Meanwhile, when an amine, a benzodioxole compound including a nitrogen atom, or the like are used as reducing agents, these can be made less likely to impair the stability by making them photolatent.

Although examples of the benzodioxole compound in the present disclosure include the following specific examples ((1-1) to (1-14)), it is not limited to these specific examples.

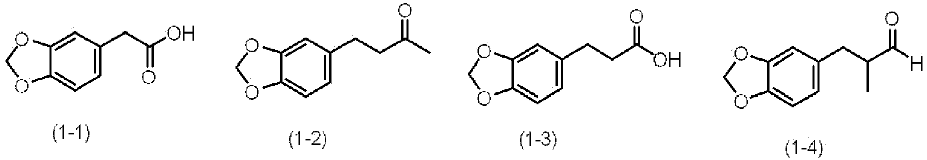

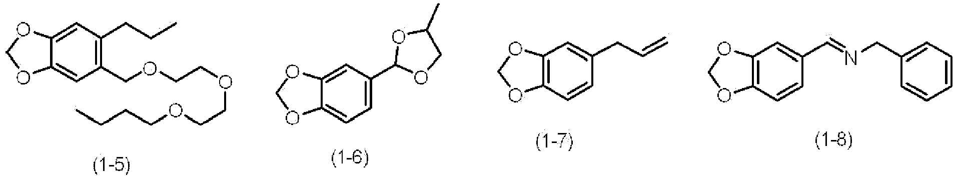

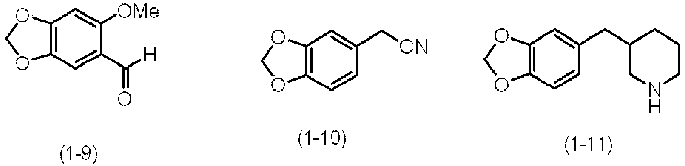

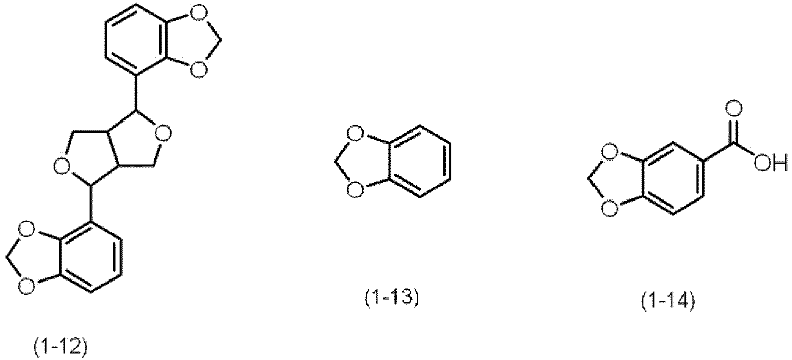

From the viewpoint of stability of a photocurable composition including the photopolymerization initiator of the present disclosure, a photoradical generator that is used in combination with a reducing agent is preferably the above Norrish type II initiator, and more preferably camphorquinone.

Furthermore, from the viewpoint of stability of a photocurable composition including the photopolymerization initiator of the present disclosure, a combination of a photoradical generator and a reducing agent is preferably a Norrish type II initiator and a benzodioxole compound, and more preferably camphorquinone and a benzodioxole compound.

When the combination of a Norrish type II initiator and a benzodioxole compound is used as a photoradical generator and a reducing agent in the photopolymerization initiator of the present disclosure, the activity to light of from near-ultraviolet region to visible region (specifically, a visible region of wavelengths of from 420 nm to 525 nm) is superior to the case in which each of these is used singly.

Therefore, when the combination of a Norrish type II initiator and a benzodioxole compound is used as a photoradical generator and a reducing agent in the photopolymerization initiator of the present disclosure, the curing rate and the depth of polymerization of a photocurable composition can be further improved, in comparison with the case in which each of a Norrish type II initiator and a benzodioxole compound is used singly.

When the combination of a Norrish type II initiator and a benzodioxole compound is used as a photoradical generator and a reducing agent in the photopolymerization initiator of the present disclosure, it is favorable as a photopolymerization initiator included in a photocurable composition that is cured by light of from near-ultraviolet region to visible region.

When the photopolymerization initiator of the present disclosure includes the above combination as a photoradical generator and a reducing agent, the applications are not particularly limited, and it is applicable to any application such as in the industrial field and the medical field.

Particularly, it is favorable as a photopolymerization initiator in a photocurable composition that is cured by light of visible region that is less influential to bodies (for example, a visible region of wavelengths of from 420 nm to 525 nm), and more specifically, it is particularly favorable as a medical photopolymerization initiator (particularly preferably as a photopolymerization initiator for a dental material).

In the photocurable composition of the present disclosure, a mass content ratio of a reducing agent to a photoradical generator (hereinafter, also referred to as a mass content ratio [reducing agent/photoradical generator]) is preferably from 0.1 to 20.0, more preferably from 0.5 to 10.0, and still more preferably from 0.7 to 1.5, from the viewpoint of obtaining a favorable depth of polymerization and from the viewpoints of preventing a reducing agent from functioning as a plasticizing agent and preventing a photoradical generator from eluting from a cured product.

A content of a reducing agent with respect to a total amount of the photopolymerization initiator of the present disclosure is preferably from 0.0001% by mass to 1.0% by mass, more preferably from 0.0002% by mass to 0.1% by mass, and still more preferably from 0.0003% by mass to 0.03% by mass.

### <Photobase Generator>

The photopolymerization initiator of the present disclosure includes a photobase generator.

The photobase generator is a compound that generates a base upon exposure to light and is capable of causing curing reactions such as polymerization reaction and condensation reaction by using the generated base as a catalyst.

As a photobase generator, for example, oxime ester compounds, acyl compounds, carbamate compounds, aminoacetophenone compounds, tertiary amine compounds, quaternary ammonium salt compounds, and amide compounds can be used. Specifically, examples thereof include triazabicyclodecene tetraphenylborate, phosphazene base Pi-t-octyl tetraphenylborate, triazabicyclodecene oxoxanthene-propionate, formyl-dimethyl-benzamide, and piperidine-carbonyl-benzoate.

Among them, because a depth of curing of a cured product is excellent, a photobase generator in the photopolymerization initiator of the present disclosure preferably includes at least one selected from the group consisting of oxime ester compounds, acyl compounds, carbamate compounds, aminoacetophenone compounds, tertiary amine compounds, quaternary ammonium salt compounds, and amide compounds, and more preferably includes at least one selected from the group consisting of acyl compounds, tertiary amine compounds, and quaternary ammonium salt compounds.

In the present disclosure, a mass content ratio of a photobase generator to a total amount of a peroxide (hereinafter, also referred to as a mass content ratio [photobase generator/peroxide]) is, from the viewpoint of obtaining a favorable depth of polymerization, preferably from 0.01 to 10.0, more preferably from 0.3 to 3.0, and still more preferably from 0.5 to 2.3.

A content of a photobase generator with respect to a total amount of the photopolymerization initiator of the present disclosure is preferably from 0.001% by mass to 1.0% by mass, more preferably from 0.01% by mass to 0.1% by mass, and still more preferably from 0.01% by mass to 0.07% by mass.

### <Peroxide>

The photopolymerization initiator of the present disclosure includes a peroxide.

Thus, in addition to the radicals generated from the above photoradical generator, radicals can be further generated from the peroxide, and a favorable depth of polymerization can be obtained.

Examples of the peroxide in the present disclosure include: diacyl peroxide compounds such as benzoyl peroxide, 2,4-dichlorobenzoyl peroxide, and m-toluoyl peroxide; peroxyester compounds such as t-butyl peroxybenzoate, bis-t-butyl peroxyisophthalate, 2,5-dimethyl-2,5-bis(benzoylperoxy)hexane, t-butyl peroxy-2-ethylhexanoate, and t-butylperoxy isopropyl carbonate; hydroperoxide compounds such as t-butyl hydroperoxide; alkyl peroxides such as dicumyl peroxide, di-t-butyl peroxide, and lauroyl peroxide; peroxyketal compounds such as 1,1-bis(t-butylperoxy)-3,3,5-trimethylcyclohexane; ketone peroxide compounds such as methyl ethyl ketone peroxide; fluorinated peroxide compounds such as perfluoro(tert-butyl peroxide), and perfluorobenzoyl peroxide; and inorganic peroxides such as potassium peroxide and hydrogen peroxide.

Among them, from the view point of obtaining a favorable depth of polymerization, the photopolymerization initiator of the present disclosure preferably includes at least one selected from the group consisting of diacyl peroxide compounds, peroxyester compounds, hydroperoxide compounds, inorganic peroxides, and alkyl peroxides, and more preferably includes at least one selected from the group consisting of diacyl peroxide compounds, hydroperoxide compounds, and inorganic peroxides.

A content of a peroxide with respect to a total amount of the photopolymerization initiator of the present disclosure is preferably from 0.001% by mass to 0.1% by mass, more preferably from 0.005% by mass to 0.07% by mass, and still more preferably from 0.01% by mass to 0.05% by mass.

### <Photocurable Composition>

The photocurable composition of the present disclosure includes the photopolymerization initiator of the present disclosure and a polymerizable monomer.

The photopolymerization initiator in the photocurable composition of the present disclosure is the same as the above photopolymerization initiator, and preferred embodiments and preferred ranges, except the following respects, are the same as those of the above photopolymerization initiator.

In the photocurable composition of the present disclosure, a total content of the photoradical generator of the present disclosure, a peroxide, a photobase generator, and a reducing agent as an optional component may be selected as appropriate in consideration of a content with respect to a polymerizable monomer as described above, or, for example, the total content may be in a range of from 0.1% by mass to 10.0% by mass with respect to a total amount of the photocurable composition.

### <Polymerizable Monomer>

The photocurable composition of the present disclosure includes a polymerizable monomer.

The polymerizable monomer included in the photocurable composition of the present disclosure may be only one kind, or may be two or more kinds.

A compound including a radical-polymerizable group is preferably used as a polymerizable monomer.

The radical-polymerizable group is preferably a group including an ethylenic double bond, and particularly preferably a (meth)acryloyl group.

The polymerizable monomer preferably includes at least one kind of a (meth)acrylate compound.

In this case, a percentage of a (meth)acrylate compound in the polymerizable monomer is preferably 60% by mass or more, more preferably 80% by mass or more, and still more preferably 90% by mass or more.

Furthermore, a percentage of a (meth)acrylate compound in the polymerizable monomer may be set at 100% by mass or less, 95% by mass or less, or 90% by mass or less.

Examples of the (meth)acrylate compound include monofunctional (meth)acrylate compounds and bifunctional or more (meth)acrylate compounds.

The (meth)acrylate compound preferably include a bifunctional or more (meth)acrylate compound, more preferably include a bifunctional to hexafunctional (meth)acrylate compound, still more preferably include a bifunctional to tetrafunctional (meth)acrylate compound, and particularly preferably include a bifunctional (meth)acrylate compound (that is, a di(meth)acrylate compound).

The bifunctional (meth)acrylate compound (that is, the di(meth)acrylate compound) is not particularly limited, and examples thereof include urethane di(meth)acrylate, neopentyl di(meth)acrylate, 1,4-butanediol di(meth)acrylate, 1,6-hexanediol di(meth)acrylate, 1,8-octanediol di(meth)acrylate, 1,9-nonanediol di(meth)acrylate, 1,10-decanediol di(meth)acrylate, tricyclodecanedimethanol di(meth)acrylate, ethyleneglycol di(meth)acrylate, diethyleneglycol di(meth)acrylate, triethyleneglycol di(meth)acrylate, tetraethyleneglycol di(meth)acrylate, polyethyleneglycol di(meth)acrylate, tripropyleneglycol di(meth)acrylate, tetrapropyleneglycol di(meth)acrylate, polypropyleneglycol di(meth)acrylate, 2,2-bis[4-(3-(meth)acryloyloxy-2-hydroxypropoxy)phenyl]propane, ethylene oxide-modified bisphenol A di(meth)acrylate, and propylene oxide-modified bisphenol A di(meth)acrylate.

A percentage of a di(meth)acrylate compound in the polymerizable monomer is preferably 60% by mass or more, more preferably 80% by mass or more, and still more preferably 90% by mass or more.

Furthermore, a percentage of a di(meth)acrylate compound in the polymerizable monomer may be set at 100% by mass or less, 95% by mass or less, or 90% by mass or less.

A percentage of a di(meth)acrylate compound in a (meth)acrylate compound is preferably 60% by mass or more, more preferably 80% by mass or more, and still more preferably 90% by mass or more.

Furthermore, a percentage of a di(meth)acrylate compound in a (meth)acrylate compound may be set at 100% by mass or less, 95% by mass or less, or 90% by mass or less.

In terms of the polymerizable monomer, reference may be made as appropriate to descriptions in publicly known documents, for example, Japanese Patent Application Laid-Open (JP-A) No. H11-315059, Japanese Patent Application Laid-Open (JP-A) No. 2001-70437, Japanese Patent Application Laid-Open (JP-A) No. 2011-105722, Japanese Patent Application Laid-Open (JP-A) No. 2000-204069, Japanese National Phase Publication (JP-A) No. 2013-544823, International Publication (WO) No. 2016/125758, and International Publication (WO) No. 2015/119163.

A content of the polymerizable monomer in the photocurable composition of the present disclosure, with respect to a total amount of the photocurable composition, is preferably 15% by mass or more, and more preferably 35% by mass or more.

Furthermore, a content of the polymerizable monomer in the photocurable composition is preferably 70% by mass or less, more preferably 45% by mass or less, and still more preferably 40% by mass or less.

A content of the photoradical generator included in the photocurable composition of the present disclosure is not particularly limited. A mass content ratio of the photoradical generator to a total amount of the polymerizable monomer (hereinafter, also referred to as a content ratio [photoradical generator/polymerizable monomer]) is preferably from 0.0001 to 1.0, more preferably from 0.0005 to 0.1, and still more preferably from 0.0005 to 0.01.

In the present disclosure, a mass content ratio of the photobase generator to a total amount of the polymerizable monomer (hereinafter, also referred to as a mass content ratio [photobase generator/polymerizable monomer]) is preferably from 0.0001 to 5.0, more preferably from 0.001 to 1.0, and still more preferably from 0.005 to 0.1.

In the present disclosure, a mass content ratio of the peroxide to a total amount of the polymerizable monomer (hereinafter, also referred to as a mass content ratio [peroxide/polymerizable monomer]) is preferably from 0.001 to 5.0, more preferably from 0.003 to 1.0, and still more preferably from 0.003 to 0.05.

### <Filler>

The photocurable composition of the present disclosure may include, if necessary, a filler.

A filler commonly used in the dental field can be used as the filler.

Fillers are usually classified broadly into organic fillers and inorganic fillers.

Examples of organic fillers include fine powders of polymethyl methacrylate, polyethyl methacrylate, methyl methacrylate-ethyl methacrylate copolymer, crosslinked polymethyl methacrylate, crosslinked polyethyl methacrylate, ethylene-vinyl acetate copolymer, and styrene-butadiene copolymer.

Examples of inorganic fillers include: fine powders of various kinds of glass, various kinds of ceramics, diatomaceous earth, kaolin, clay minerals (such as montmorillonite), activated white clay, synthetic zeolite, mica, calcium fluoride, ytterbium fluoride, calcium phosphate, barium sulfate, zirconium dioxide, titanium dioxide, and hydroxyapatite; and silica, alumina, zirconia and titania.

The above various kinds of glass include silicon dioxide as a main component, and, if necessary, may include oxides of a heavy metal, boron, aluminum and the like.

Examples of the various kinds of glass include barium borosilicate glass, aluminosilicate glass (such as boroaluminosilicate glass, strontium boroaluminosilicate glass, fluoroaluminosilicate glass).

Specific examples of such inorganic fillers include barium borosilicate glass (such as Kimble Raysorb T3000, Schott 8235, Schott GM27884, and Schott GM39923), strontium boroaluminosilicate glass (such as Raysorb T4000, Schott G018-093, and Schott GM32087), lanthanum glass (such as Schott GM31684), fluoroaluminosilicate glass (such as Schott G018-091 and Schott G018-117), and boroaluminosilicate glass including such as zirconium and cesium (such as Schott G018-307, G018-308, and G018-310).

Further, it is possible to use an organic-inorganic composite filler that is obtained through adding a polymerizable monomer to an inorganic filler in advance, forming the mixture into a paste, and then curing the resultant by polymerization, followed by crushing.

Furthermore, in the photocurable composition, an embodiment in which a microfiller having a particle size of 0.1 µm or less is included is one of the favorable embodiments for a photocurable composition for a dental material.

Such a filler having a small particle size is preferably made of a material such as silica (for example, AEROSIL (brand name)), alumina, zirconia, or titania.

Among them, as a filler, it is preferable to include at least one filler selected from the group consisting of silica, barium borosilicate glass, and aluminosilicate glass.

These fillers may be surface treated by a surface treatment agent such as a silane coupling agent, depending on the purpose. Examples of the surface treatment agent include known silane coupling agents, for example, organosilicon compounds such as methacryloxyalkyltrimethoxysilane (the number of carbon atoms between the methacryloxy group and the silicon atom: 3 to 12), methacryloxyalkyltriethoxysilane (the number of carbon atoms between the methacryloxy group and the silicon atom: 3 to 12), vinyltrimethoxysilane, vinylethoxysilane and vinyltriacetoxysilane.

From the viewpoint of improving strength of a cured product, a content of the filler in the photocurable composition of the present disclosure with respect to a total amount of the photocurable composition is preferably 30% by mass or more, more preferably 40% by mass or more, and still more preferably 50% by mass or more.

Furthermore, from the viewpoint of suppressing a decrease in toughness of the cured product and viscosity of the composition, a content of the filler in the photocurable composition is preferably 80% by mass or less, more preferably 75% by mass or less, and still more preferably 65% by mass or less.

The photocurable composition of the present disclosure preferably further includes a filler having a relative permittivity (εₛ) of 8.0 or less at 25°C and 1 MHz (hereinafter, also referred to as a specific filler).

A relative permittivity (εₛ) of the specific filler at 25°C and 1 MHz is more preferably 6.5 or less.

A lower limit of a relative permittivity (εₛ) of the specific filler at 25°C and 1 MHz is not particularly limited, and it may be 0.1 or more, or may be 1.0 or more.

Examples of the filler having a relative permittivity (εₛ) of 8.0 or less at 25°C and 1 MHz include silica, barium borosilicate glass, and aluminosilicate glass (such as boroaluminosilicate glass, strontium boroaluminosilicate glass, fluoroaluminosilicate glass).

The relative permittivity (εₛ) in the present disclosure is measured under the condition of 25°C and 1 MHz, based on the paragraphs 2.5.5.9 of IPC-TM-650, which is a test manual of the IPC standards.

As a measuring apparatus, for example, the impedance material analyzer HP4291A can be used.

In the present disclosure, a relative permittivity of a filler means a relative permittivity of a component that is included in a photocurable composition as a filler (in the present specification, also referred to as a filler component).

When a filler is formed with the filler component and then a relative permittivity of the formed filler is measured, the measured relative permittivity value becomes lower in some cases due to the influence of voids in the formed filler.

A relative permittivity of the filler in the present disclosure is obtained by measuring a relative permittivity of a component that is included in the photocurable composition as a filler, for the purpose of excluding the influence of voids in the formed filler. Therefore, a measurement sample is not necessarily in a shape of a filler, as long as it is composed of the same component as that in a filler to be measured.

Specific examples of the component included in a photocurable composition as a filler include the above organic filler and inorganic filler.

A total content of the specific filler with respect to a total amount of the filler is preferably 50% by mass or more, more preferably 70% by mass or more, and still more preferably 90% by mass or more.

### <Photosensitizer>

The photocurable composition of the present disclosure may include a photosensitizer.

A photosensitizer in the present disclosure means a component that facilitates radical generation of a photoradical generator by propagating energy obtained through light absorption by itself to the above photoradical generator.

Examples of the photosensitizer in the present disclosure include: naphthalene compounds such as diethoxynaphthalene; anthracene compounds such as dibutoxyanthracene; thioxanthone compounds such as isopropylthioxanthone; and coumarin compounds such as carbonylbis(diethylaminocoumarin) and benzimidazolyl dimethylaminocoumarin.

Among them, from the viewpoint of achieving a favorable depth of polymerization, naphthalene compounds, anthracene compounds, thioxanthone compounds, and coumarin compounds are preferable.

In the present disclosure, a mass content ratio of the photosensitizer to a total amount of the polymerizable monomer (hereinafter, also referred to as a mass content ratio [photosensitizer/polymerizable monomer]) is, from the viewpoint of obtaining a favorable depth of polymerization, preferably from 0.0003 to 0.07, more preferably from 0.0005 to 0.05, and still more preferably from 0.001 to 0.035.

### <Other Components>

The photocurable composition of the present disclosure may include, if necessary, components other than the above components.

Examples of the other components include a polymerization inhibitor, a colorant (for example, a pigment or a dye), a reinforcing material (for example, a fiber), a bactericide, a disinfectant, a stabilizer, and a preservative agent.

In terms of other components, reference may be made as appropriate to descriptions in publicly known documents, for example, Japanese Patent Application Laid-Open (JP-A) No. H11-315059, Japanese Patent Application Laid-Open (JP-A) No. 2001-70437, Japanese Patent Application Laid-Open (JP-A) No. 2011-105722, Japanese Patent Application Laid-Open (JP-A) No. 2000-204069, Japanese National Phase Publication (JP-A) No. 2013-544823, International Publication (WO) No. 2016/125758, and International Publication (WO) No. 2015/119163.

A cured product of the photocurable composition can be obtained by performing light irradiation (preferably visible light irradiation) using publicly known means to the photocurable composition of the present disclosure.

Mechanical property of the cured product may be improved by further performing heat treatment, after light irradiation, to the photocurable composition of the present disclosure.

### <Applications of Photopolymerization Initiator and Photocurable Composition>

Applications of the photopolymerization initiator and the photocurable composition of the present disclosure are not particularly limited.

The photopolymerization initiator and the photocurable composition of the present disclosure can be used, for example, as a paint, a composition for coating film formation, a dental material, and a component in a dental material.

The photopolymerization initiator and the photocurable composition of the present disclosure are preferably used as dental materials or components in a dental material. In other words, the photopolymerization initiator of the present disclosure is preferably a photopolymerization initiator for a dental material, and the photocurable composition of the present disclosure is preferably a photocurable composition for a dental material.

### [Cured Product]

The cured product of the present disclosure is a cured product of the above photocurable composition of the present disclosure or the above dental material of the present disclosure.

Therefore, the cured product of the present disclosure is excellent in a depth of polymerization.

The cured product of the present disclosure is obtained by performing light irradiation (preferably visible light irradiation) to the above photocurable composition of the present disclosure or the above dental material of the present disclosure.

Mechanical property of the cured product may be improved by further performing heat treatment after light irradiation.

### [Dental Material]

The dental material of the present disclosure consists of the cured product of the photocurable composition of the present disclosure described above.

The dental material of the present disclosure is a cured product excellent in a depth of polymerization.

The dental material of the present disclosure consists of the above cured product. This enables dental restoration with an excellent depth of polymerization.

Examples of the composition for a dental material and the dental material of the present disclosure include a dental restorative composite resin, a denture base resin, a denture base lining material, an impression material, a cementing material (such as a resin cement and a resin-modified glass ionomer cement), a dental adhesive (such as an orthodontic adhesive and a cavity coating adhesive), a tooth fissure sealant, a resin block for CAD/CAM, a temporary crown, and an artificial tooth material.

Examples of the dental restorative composite resin include a composite resin for a crown, a composite resin for filling a caries cavity, a composite resin for abutment construction, and a composite resin for filling and restoring.

As the composition for a dental material and the dental material of the present disclosure, a dental restorative composite resin is particularly preferred.

As described above, the cured product excellent in a depth of polymerization can be obtained by curing the composition for a dental material of the present disclosure.

Therefore, dental restoration with an excellent depth of polymerization can be performed when the composition for a dental material and the dental material of the present disclosure is a dental restorative composite resin. In other words, an excellent curing performance (particularly, a curing performance for light irradiation of visible region) is provided, through a surface of the dental restorative composite resin that has been filled in a defect of a tooth to a deep part thereof.

### EXAMPLES

Hereinafter, the present disclosure will be explained in more detail by reference to Examples. However, the present disclosure is not limited to the following Examples as long as the gist is maintained. The term "parts" is on a mass basis, unless otherwise noted,

Polymerizable monomers and photopolymerization initiators used in Examples of the present disclosure will be explained below.

### <Polymerizable Monomer>

The followings were prepared as polymerizable monomers.
UDMA: dimethacryloxyethyl trimethylhexyl dicarbamate (urethane dimethacrylate)
TEGDMA: triethyleneglycol dimethacrylate

### <Photosensitizer>

The followings were prepared as photosensitizers.
ITX: isopropylthioxanthone
UVS-1331: dibutoxyanthracene
UVS-2171: diethoxynaphthalene
KCD: carbonylbis(diethylaminocoumarin)
C7: benzimidazolyl dimethylaminocoumarin

### <Peroxides>

The followings were prepared as peroxides.
BPO: benzoyl peroxide
POSNH₄: ammonium peroxodisulfate
CHP: cumene hydroperoxide

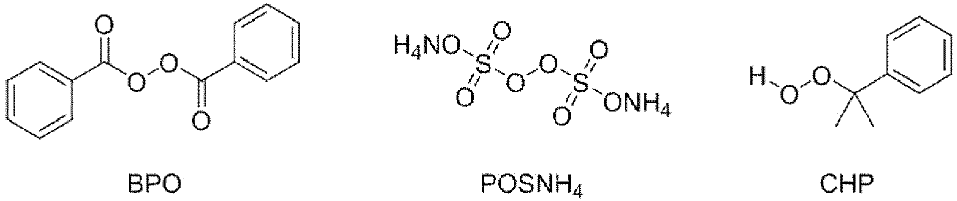

### <Photobase Generators>

The followings were prepared as photobase generators.
TBD·BPh₄: triazabicyclodecene tetraphenylborate
PN·BPh₄: phosphazene base Pi-t-octyl tetraphenylborate
O0396: triazabicyclodecene oxoxanthene-propionate
FDB: formyl-dimethyl-benzamide
PCB: piperidine-carbonyl-benzoate

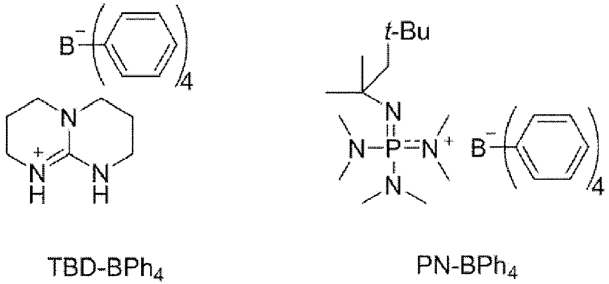

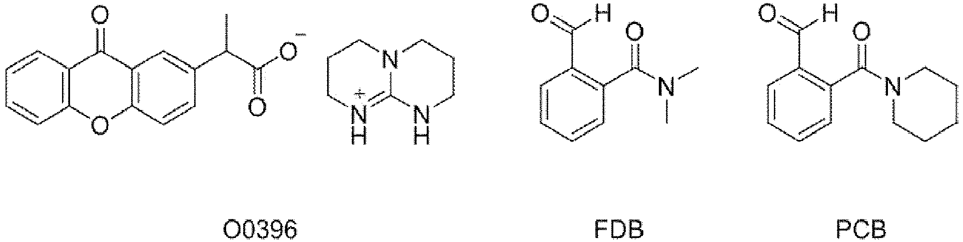

### <Photoradical Generators>

The followings were prepared as photoradical generators.
CQ: camphorquinone (a Norrish type II initiator, a photoradical generator that is excited by light having a wavelength of from 400 nm to 500 nm)

### <Reducing Agents for Photoradical Generators>

BDO: benzodioxole
PB: piperonyl butoxide
EDB: ethyl dimethylamino benzoate

### <Filler>

The following was prepared as a filler.
Filler: barium borosilicate glass (0.85 µm, treated with silane coupling, the relative permittivity (εₛ) at 25°C and 1 MHz: 6.2)

The above relative permittivity was measured by the method described earlier.

### [Examples 1 to 14 and Comparative Examples 1 to 5]

### <Preparation of Photocurable Compositions>

The components shown in the following Table 1 were weighted and put in a light-shielding bottle, followed by stirring for 2 minutes under the condition of 20 kPa and 2000 rpm (revolutions per minutes) using a planetary centrifugal mixer manufactured by THINKY corporation to obtain each of the photocurable compositions of Examples 1 to 14 and Comparative Examples 1 to 5.

The number in a column of each component shown in Table 1 means the amount (parts by mass) of each component.

The blank columns in Table 1 means the components were not used.

### <Stability Evaluation>

Stability evaluation was performed as follows for each of the photocurable compositions of Examples 1 to 14 and Comparative Examples 1 to 5.

A photocurable composition was put in a light-shielding bottle, and after 24 hours, the composition in the container was stirred using a spatula to examine whether a gel-like hardened material without fluidity was observed or not. The results were shown in Table 1.

In the following evaluation criteria, a grade of the most excellent stability is "A".

### - Evaluation Criteria of the Stability -

A: fluidity is favorable, and no gel-like hardened material was observed.

B: fluidity was observed, and a gel-like hardened material was observed at approximately half of the total volume of a photocurable composition.

### <Depth of Polymerization Evaluation>

A depth of polymerization was evaluated as follows for each of the photocurable compositions of Examples 1 to 14 and Comparative Examples 1 to 5.

A photocurable composition was put in a glass tube of 10 mm × 75 mm, and was irradiated for 20 seconds using a light irradiation apparatus Translux 2Wave manufactured by Kulzer. Subsequently, the resultant was left to stand for 96 hours under a light-shielded condition, followed by measuring a length of a cured product retrieved from the glass tube.

A depth of polymerization was evaluated based on the following evaluation criteria (grades).

The results were shown in Table 1.

In the following evaluation criteria, a grade of the most excellent depth of polymerization is "A".

### - Evaluation Criteria of the Depth of Polymerization -

A: a depth of polymerization was 40 mm or more.
B: a depth of polymerization was less than 40 mm, but 35 mm or more.
C: a depth of polymerization was less than 35 mm, but 30 mm or more.
D: a depth of polymerization was less than 30 mm, but 25 mm or more.
E: a depth of polymerization was less than 25 mm.

**Table 1**

| | | Examples | | | | | | | | | | | | | | Comparative Examples | | | | |
|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 1 | 2 | 3 | 4 | 5 |
| Polymerizable monomer | UDMA | 3.126 | 3.119 | 3.119 | 3.115 | 3.117 | 3.797 | 3.123 | 3.934 | 3.120 | 3.129 | 3.913 | 3.129 | 3.923 | 3.906 | 3.134 | 3.185 | 3.859 | 3.870 | 3.964 |
| | TEGDMA | 0.800 | 0.800 | 0.800 | 0.800 | 0.800 | | 0.800 | | 0.800 | 0.800 | | 0.800 | | | 0.800 | 0.800 | | | |
| Photosensitizer | ITX | 0.005 | 0.005 | | | | | 0.005 | | 0.005 | 0.005 | | | | | 0.005 | | | | |
| | UVS-1331 | | | 0.006 | | | 0.060 | | 0.006 | | | 0.006 | | 0.006 | 0.006 | | | | | |
| | UVS-2171 | | | | | | 0.060 | | 0.006 | | | 0.006 | | 0.006 | 0.006 | | | | | |
| | KCD | | | | 0.010 | | | | | | | | | | | | | | | |
| | C7 | | | | | 0.007 | | | | | | | | | | | | | | |
| Peroxide | BPO | 0.025 | 0.025 | 0.025 | 0.025 | 0.025 | 0.025 | 0.025 | 0.025 | | | 0.025 | 0.025 | 0.025 | 0.025 | 0.025 | | | 0.025 | 0.025 |
| | POSNH4 | | | | | | | | | 0.025 | | | | | | | | | | |
| | CHP | | | | | | | | | | 0.016 | | | | | | | | | |
| Photobase generator | TBD·BPh4 | 0.036 | 0.036 | 0.036 | 0.036 | 0.036 | | | | 0.036 | 0.036 | 0.036 | 0.036 | 0.036 | 0.036 | 0.036 | | 0.036 | | |
| | PN·BPh4 | | | | | | 0.043 | | | | | | | | | | | | | |
| | O0396 | | | | | | | 0.032 | | | | | | | | | | | | |
| | FDB | | | | | | | | 0.014 | | | | | | | | | | | |
| | PCB | | | | | | | | | | | | | | 0.017 | | | | | |
| photoradical generator | CQ | 0.005 | 0.005 | 0.005 | 0.005 | 0.005 | 0.005 | 0.005 | 0.005 | 0.005 | 0.005 | 0.005 | 0.005 | 0.005 | 0.005 | | 0.005 | 0.005 | 0.005 | 0.005 |
| Reducing agent | BDO | 0.004 | | | | | | | | | | | | | | | | | | |
| | PB | | 0.010 | 0.010 | 0.010 | 0.010 | 0.010 | 0.010 | 0.010 | 0.010 | 0.010 | 0.010 | | | | | 0.010 | 0.100 | 0.100 | |
| | EDB | | | | | | | | | | | | 0.006 | | | | | | | 0.006 |
| Filler | Filler | 6.000 | 6.000 | 6.000 | 6.000 | 6.000 | 6.000 | 6.000 | 6.000 | 6.000 | 6.000 | 6.000 | 6.000 | 6.000 | 6.000 | 6.000 | 6.000 | 6.000 | 6.000 | 6.000 |
| Stability | | A | A | A | A | A | A | A | A | A | A | A | B | A | A | A | A | A | A | A |
| Depth of polymerization (mm) | | 35 | 30 | 35 | 75 | 75 | 75 | 35 | 75 | 30 | 70 | 75 | 75 | 75 | 75 | 2 | 5 | 5 | 5 | 9 |
| Depth of polymerization (grade) | | B | C | B | A | A | A | B | A | C | A | A | A | A | A | E | E | E | E | E |

As shown in Table 1, the depth of polymerization was excellent in Examples 1 to 14.

Among them, the depth of polymerization was excellent in Examples 4 and 5 where the coumarin derivative was used as the photosensitizer, and in Examples 6, 8, and 11 where the anthracene derivative and the naphthalene derivative were used in combination as the photosensitizer.

Although it is not shown in Table 1, in Examples 6, 8, and 11 where the anthracene derivative and the naphthalene derivative were used in combination, coloring of cured products were suppressed compared with those in Examples 4 and 5 where the coumarin derivative was used.

Further, in each Examples 1 to 11 where benzodioxole or its derivative, but not amine, was used as the reducing agent, the stability of each of them was excellent compared with that in Example 12 where amine was used as the reducing agent.

Furthermore, the depth of polymerization was excellent in Example 10 where the hydroperoxide was used as the peroxide.

On the other hand, the depth of polymerization was poor in each of Comparative Example 1 where a photoradical generator was not included, Comparative Example 2 where a photobase generator and a peroxide were not included, Comparative Example 3 where a peroxide was not included, and Comparative Examples 4 and 5 where the photobase generators were not included.

The disclosures of Japanese Patent Application No. 2019-007972, filed January 21, 2019, and Japanese Patent Application No. 2019-064404, filed March 28, 2019, are incorporated herein by reference in their entirety.

All publications, patent applications, and technical standards mentioned in this specification are herein incorporated by reference to the same extent as if each individual publication, patent application, or technical standard was specifically and individually indicated to be incorporated by reference.

## Claims

1. A photopolymerization initiator, comprising:
a peroxide;
a photobase generator; and
a photoradical generator.

2. The photopolymerization initiator according to claim 1, wherein the photoradical generator is excited by light having a wavelength of from 420 nm to 525 nm.

3. The photopolymerization initiator according to claim 1 or 2, wherein the photoradical generator is a Norrish type II initiator, or an aniline compound and an iodonium salt compound.

4. The photopolymerization initiator according to any one of claims 1 to 3, wherein the peroxide comprises at least one selected from the group consisting of diacyl peroxide compounds, peroxyester compounds, hydroperoxide compounds, inorganic peroxides, and alkyl peroxides.

5. The photopolymerization initiator according to any one of claims 1 to 4, wherein the photobase generator comprises at least one selected from the group consisting of oxime ester compounds, acyl compounds, carbamate compounds, aminoacetophenone compounds, tertiary amine compounds, quaternary ammonium salt compounds, and amide compounds.

6. The photopolymerization initiator according to any one of claims 1 to 5, further comprising a benzodioxole compound.

7. The photopolymerization initiator according to any one of claims 1 to 6, which is for a dental material.

8. A photocurable composition, comprising:
the photopolymerization initiator according to any one of claims 1 to 7; and
a polymerizable monomer.

9. The photocurable composition according to claim 8, wherein a mass content ratio of the photoradical generator to a total amount of the polymerizable monomer is from 0.0001 to 1.0.

10. The photocurable composition according to claim 8 or 9, wherein a mass content ratio of the photobase generator to a total amount of the polymerizable monomer is from 0.0001 to 5.0.

11. The photocurable composition according to any one of claims 8 to 10, wherein a mass content ratio of the peroxide to a total amount of the polymerizable monomer is from 0.001 to 5.0.

12. The photocurable composition according to any one of claims 8 to 11, further comprising a filler.

13. The photocurable composition according to claim 12, wherein the filler comprises a filler having a relative permittivity of 8.0 or less at 25°C and 1 MHz.

14. The photocurable composition according to claim 12 or 13, wherein the filler comprises at least one filler selected from the group consisting of silica, barium borosilicate glass, and aluminosilicate glass.

15. The photocurable composition according to any one of claims 8 to 14, which is for a dental material.

16. A cured product of the photocurable composition according to any one of claims 8 to 14.

17. A dental material, comprising the cured product according to claim 16.
